# EUROPEAN PATENT SPECIFICATION

(11) **EP 2 885 048 B1**
(45) Date of publication and mention of the grant of the patent: **27.09.2017**
(21) Application number: 13753469.9
(22) Date of filing: 15.08.2013
(51) Int. Cl.: A61M 39/08, B32B 27/30, B32B 27/40, B32B 1/08

(54) **POLYURETHANE-POLYETHYLENE DELAMINATION RESISTANT TUBING WITH GAS BARRIER PROPERTIES**
ABLÖSUNGSRESISTENTES POLYURETHAN-/POLYETHYLENROHR MIT GASBARRIEREEIGENSCHAFTEN
TUBAGE EN POLYURÉTHANNE-POLYÉTHYLÈNE RÉSISTANT À LA DÉLAMINATION DOTÉ DE PROPRIÉTÉS DE BARRIÈRE VIS-À-VIS DES GAZ

(30) Priority: 15.08.2012 US 201213586288
(43) Date of publication of application: 24.06.2015
(73) Proprietor: Tekni-Plex, Inc., King of Prussia, PA 19406 (US)
(72) Inventor: BOURGEOIS, Philip, Perrysburg, OH 43551 (US); MUNISH, Shah, Sylvania, OH 43560 (US)
(74) Representative: Jenkins, Peter David
(86) International application number: PCT/US2013/055075
(87) International publication number: WO 2014/028700

(56) References cited:
- EP-A1- 0 244 960
- WO-A1-97/41906
- DE-U1-202004 000 533
- US-A1- 2009 087 606

## Description

### Field of the Invention

The present invention relates to polymeric tubing typically formed by a co-extrusion process, the tubing having multiple layers of the same or different polymeric materials, each layer successively adhered to each other.

### Background

Tubing comprised of polymeric material is used in many industrial and commercial applications including in the medical field. Various FDA compliant plastics are used, depending upon properties desired and the intended applications. Where the tubing is used to transport fluids for in vivo treatment of human patients, selection of the polymeric materials can be a factor.

Polyvinyl chloride (PVC) is one of the most widely used plastics. While structurally stable and easily formable into desired shapes, PVC is typically manufactured using plasticizers which can migrate out of the PVC matrix into bodily fluids and has other properties not ideally suited for medical treatment applications. Likewise, due to the inherent nature of plasticized PVC tubing, there arises the potential absorption of medicines and other components of aqueous fluids used in medical treatments into the sidewall of the PVC tube. Polyurethane is potentially a substitute for PVC. However, dual layer tubing comprised of polyurethane and polyethylene suffers from the inability of the two layers to remain adhered to each other under low to moderate stress, strain or mechanical manipulation conditions as well as the inability to sufficiently impede migration of oxygen through the layers. U.S. Pat. No. 4,627,844 to Schmitt ("Schmitt"), the disclosure of which is related to the subject matter herein, discloses a tri-layer tube which is embodied in a commercial product sold under the trademark "SUREPATH 151" by the Natvar Division of Tekni-Plex, Inc. As disclosed in Schmitt, an outer layer of PVC and an inner fluid-contact layer of low density polyethylene (LDPE) are co-extruded with an intermediate tie layer of ethylene vinyl acetate copolymer (EVA). However, while Schmitt greatly reduces the possibility for the migration of additives from the PVC to the fluid and absorption of components from the fluid to the PVC tubing by providing a LDPE fluid-contact layer, elimination of the PVC is preferred. Other tubing configurations are disclosed in U.S. Patent No. 7,647,949, U.S. Patent No. 4,211,741 and U.S. Patent Publication No. 2007/0119511, the disclosures of which are related to the subject matter herein. Where medical tubing is concerned, preservation of the integrity of reagents contained in fluids being routed through the tubing can be a concern. Similarly, prevention of migration of components out of the fluids through the tubing can be an issue. In such applications, incorporation into the tubing of a layer of material comprised of a gas barrier material can be implemented for purposes of preventing migration of gases such as oxygen into the fluid thus preserving oxygen sensitive reagents in the fluid. US2009/087606 discloses various tube structures comprising an inner-most layer, an outer-most layer, and a barrier layer.

### Summary of the Invention

In accordance with the invention there is provided a tube according to claim 1.

The adhesive typically comprises one or more anhydride grafted ethylene acrylate copolymers and preferably one or more anhydride grafted ethylene methyl acrylate copolymers.

The inner layer typically comprises more than 90% by weight of a polyethylene and the outer layer comprises more than 90% by weight of an aromatic or aliphatic polyether based polyurethane.

The adhesive typically comprises more than 90% by weight of one or more ethylene acrylic copolymers.

The inner layer can comprise more than 90% by weight of a polyethylene and the outer layer can comprise more than 90% by weight of an aromatic or aliphatic polyether based polyurethane.

The adhesive can comprise more than 90% by weight of one or more ethylene acrylic copolymers and the inner layer can comprise more than 90% by weight of a polyethylene and the outer layer can comprise more than 90% by weight of an aromatic or aliphatic polyether based polyurethane.

The polyethylene typically comprises one or more of a low density polyethylene, a linear low density polyethylene and a high density polyethylene and the aromatic polyether based polyurethane can comprise a polytetramethyleneglycol-based polyurethane.

The adhesive can comprise more than 90% by weight of one or more ethylene acrylic copolymers, the inner layer can comprise more than 90% by weight of low density polyethylene (LDPE), and the outer layer can comprise more than 90% by weight of a polytetramethyleneglycol-based polyurethane.

The adhesive typically comprises more than 90% by weight of one or more ethylene acrylic copolymers.

The thickness of the polyurethane outer layer is typically between 0.001 inches (0.0254 mm) and 0.025 inches (0.635 mm), the thickness of the inner polyethylene layer is typically between between 0.001 inches (0.0254 mm) and 0.025 inches (0.635 mm) and the thickness of the barrier layer is typically between 0.001 inches (0.0254 mm) and 0.025 inches (0.635 mm).

The inner and outer layers preferably do not visually delaminate when subjected to a stress and strain up to the tube's elastic yield point as measured in a mechanical tester at a pull rate of about 12 inches (305 mm) per minute at ambient conditions of 72 degrees F (22° C) and 50% relative humidity.

Preferably, the tube does not visually delaminate when submersed in water at 60° C for 36 hours.

The tube preferably has a central axial fluid flow passage through which aqueous fluid is routed, the inner layer having a radially inner wall surface that contacts the aqueous fluid the outer and inner layers resisting delamination when subjected to a stress and strain up to the tube's elastic yield point as measured in a mechanical tester at a pull rate of about 12 inches (305 mm) per minute at ambient conditions of 72 degrees F (22° C) and 50% relative humidity. Such a tube preferably does not visually delaminate after being submersed in water at 60° C for 36 hours.

In such a tube, the thickness of the adhesive disposed between the barrier layer and the outer layer is preferably between 0.001 inches (0.0254 mm) and 0.025 inches (0.635 mm) and the thickness of the adhesive disposed between the barrier layer and the inner layer is preferably between 0.001 inches (0.0254 mm) and 0.025 inches (0.635 mm). In another aspect of the invention there is provided, a method of forming a medical tube according to claim 11.

In such a method, the fourth polymeric material preferably is selected such that the medical tubing does not visually delaminate after being submersed in water at 60° C for 36 hours.

In such a method, the adhesive typically comprises one or more anhydride grafted ethylene acrylate copolymers.

### Brief Description of the Drawings

The drawings depict one or more embodiments of the invention that are shown by way of examples of the invention wherein:
Fig. 1 is a schematic perspective view of a five-layered tube showing the outer and middle or intermediate layers broken away in order to better illustrate the construction and arrangement of the tubing;
Fig. 2 is a cross-sectional view taken along lines 2-2 of the tube 10 shown in Fig. 1.

### Detailed Description

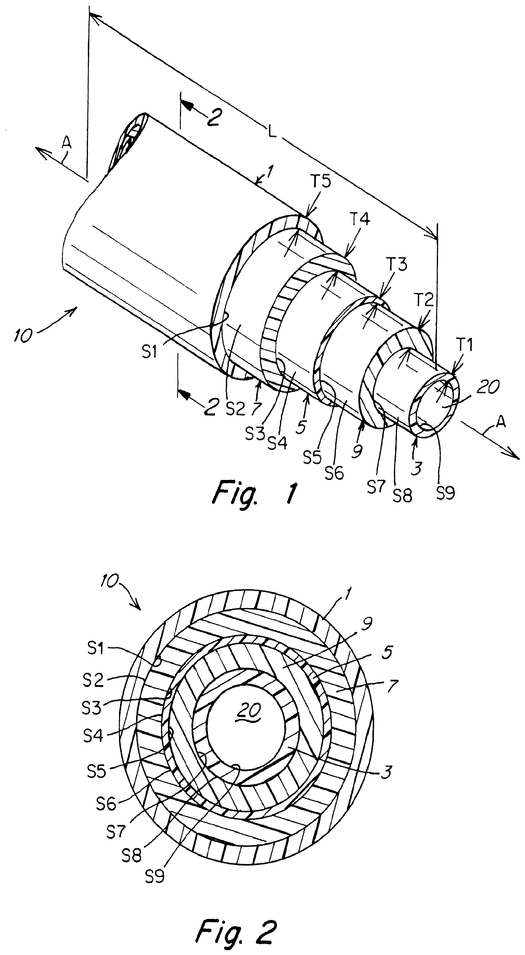

There is shown in FIG. 1 an embodiment of a co-extruded five-layer tubing 10 according to the invention which comprises an outer layer 1 comprised of at least 90% by weight of a polyurethane material, typically a polytetramethyleneglycol-based polyurethane one example of which is Lubrizol TPU Pellethane 2363-90AE, an inner fluid-contact layer 3 comprised of at least 90% by weight of a polyethylene material, typically a low density polyethylene, one example of which is Westlake LDPE EM808AA, an intermediate gas barrier layer 5 comprised of at least 90% by weight of an ethylene vinyl alcohol copolymer (EVOH), a polyamide or a mixture or blend of two or more thereof and bonding layers 7, 9 comprised of an adhesive material that bonds the barrier layer 5 to the outer 1 and inner 3 layers. The gas barrier layer 5 acts as a barrier to gases generally such as oxygen, nitrogen, hydrogen, chlorine, nitrous oxide and the like. The adhesive layers 7, 9 preferably comprise a material that renders the tubing 10 subsequent to extrusion resistant to delamination where the tubing does not visually delaminate after being subjected to submersion in water at 60° C for 36 hours and subsequently mechanically flattened by manual squeezing of the tube from its normal round in cross-sectional condition to a flattened or oval shape cross-sectional shape or condition. The adhesive material is selected to comprise one or more ethylene acrylic copolymers, an example of which is an anhydride grafted ethylene methyl acrylate copolymer, a specific example of which is an anhydride grafted ethylene methyl acrylate copolymer such as commercially available Westlake Tymax GA 7001 (Anhydride grafted Ethylene Methyl Acrylate Copolymer).

As shown in Fig. 1 the outer layer of polyurethane 1 has a radially inner facing surface S1 that binds and adheres to a radially outer facing surface S2 of the anhydride modified acrylate adhesive layer 7. The adhesive layer 7 has a radially inner facing surface S3 that binds to the radially outer facing surface S4 of the barrier layer 5. The barrier layer 5 has a radially inner facing surface S5 that binds to the radially outer facing surface S6 of another layer 9 of adhesive. The adhesive layer 9 has a radially inner facing surface S7 that binds to the radially outer facing surface S8 of the inner polyethylene layer 3. The intermediate barrier layer 5 adheres to the outer 1 and inner 3 layers such that the three layers 1, 3 and 5 remain adhered to layers 7, 9 and to each other when the tube 10 is subjected to a stress and strain up to the tube's elastic yield point as measured in a mechanical tester at a pull rate of 12 inches (305 mm) per minute at ambient conditions of 72 degrees F (22° C) and 50% relative humidity. Mechanical testers for measuring such stress and strain are knowing in the art, an example of which is a Lloyd LR5K Plus mechanical tester. Elastic yield point is the highest point at which one or more of the layers of the tubing permanently deforms or as otherwise defined in *"*Introduction to Physical Polymer Science, 4th Edition," L.H. Sperling (author), John Wiley & Sons (publisher), 2006, the disclosure of which is related to subject matter herein.

The layers 1, 3, 5, 7, 9 of such tubing 10 remain adhered to each other such that the layers do not visually delaminate after being subjected to submersion in water at 60° C for 36 hours and subsequently mechanically flattened by manual squeezing of the tube from its normal round in cross-sectional condition to a flattened or oval shape cross-sectional shape or condition.

As shown in Figs. 1 and 2, the layers 1, 3, 5 are formed into structurally stable walls that surround and enclose a central hollow fluid passage 20 through which an aqueous solution is routed and flows along an axial A direction contacting the radially inner facing surface S9 of the inner layer 3. The adhesive layers 7, 9 bind and hold the structural layers, inner 3, intermediate 5 and outer 1 together.

The inner layer 3 provides a radially inner fluid-contact surface S9, the thickness, of the inner layer 3 typically ranging in cross-sectional thickness T1 of between 0.001 inches (0.0254 mm) and 0.025 inches (0.635 mm). The intermediate layer 5 typically ranges in cross-sectional thickness T3 of between 0.001 inches (0.0254 mm) and 0.025 inches (0.635 mm). The outer layer 1 typically ranges in cross-sectional thickness T5 of between 0.001 inches (0.0254 mm) and 0.025 inches (0.635 mm). The adhesive layers 7, 9 typically range in cross-sectional thickness T2, T4 of between 0.001 inches (0.0254 mm) and 0.025 inches (0.635 mm).

The polyethylene material is preferably a branched low-density polyethylene (LDPE), such as Westlake EM808, available from Westlake Chemical Corporation. The polyethylene material can be a linear low density polyethylene (LLDPE) such as Dowlex 2035G, available from the Dow Chemical Company. The polyethylene material can also be a high-density polyethylene (HDPE), such as Chevron 9506 HDPE, Chevron 9406 HDPE, and Chevron 9503 HDPE, available from Chevron Corporation. The polyethylene material can be a mixture or blend of two or more of the aforementioned polyethylene materials.

The polyurethane elastomer (TPU) is typically the reaction product of a polyol and isocyanate and usually includes a combination of hard and soft segment domains. An aromatic polyether-based TPU or an aliphatic polyether-based TPU can be used such as a polytetramethyleneglycol-based polyurethane. Such examples of these TPU's include the Pellethane 2363-90 AE series available from the Lubrizol Corporation.

The respective thickness of each layer of tubing 10 can be controlled by conventional multi-layer extrusion tooling and equipment and typically includes a die set configured for producing multi-layer tubing such as a five-layer tube as shown in Fig. 1. Such a suitable extrusion apparatus is selected so as to provide a uniform thickness of the layers 1, 3, 5, 7, 9 along the substantial entirety of the axial length L of all of the layers 1, 3, 5, 7, 9.

The polymeric materials of which the layers 1, 3, 5, 7, 9 are comprised are preferably selected so as to be manually flexible along and around the axis A of the tubing. The polymeric materials are also selected so as to maintain the integrity of the tubing 10 (namely delamination does not occur) and its transparency or clarity after being subjected to ethylene oxide (EtO) and gamma irradiation sterilization processes.

The foregoing description is intended to illustrate and not limit the scope of the invention, those skilled in the art will realize that changes and modifications may be made thereto without departing from the invention, all such changes and modifications falling within the scope of the claims hereof.

## Claims

1. A tube (10) comprising an inner layer (3), an outer layer (1) and a barrier layer (5) disposed between the inner layer (3) and the outer layer (1), wherein the barrier layer (5) is bound to the outer layer (1) by a layer of adhesive (7) disposed between the outer layer (1) and the barrier layer (5), wherein the inner layer (3) comprises a polyethylene, the outer layer (1) comprises a thermoplastic polyurethane and the barrier layer (5) comprises a material that acts as a barrier to gas, **characterized in that** the barrier layer (5) is bound to the inner layer (3) by a layer of adhesive (9) disposed between the inner layer (3) and the barrier layer (5), the barrier layer (5) comprises more than 90% by weight of an ethylene vinyl alcohol copolymer or a polyamide or blends thereof, and the adhesive of the layers of adhesive (7, 9) comprises one or more ethylene acrylic copolymers.

2. The tube (10) of claim 1 wherein the adhesive comprises one or more anhydride grafted ethylene acrylate copolymers, optionally wherein the adhesive comprises one or more anhydride grafted ethylene methyl acrylate copolymers, or alternatively wherein the adhesive comprises more than 90% by weight of one or more ethylene acrylic copolymers.

3. The tube (10) of claim 1 where the inner layer (3) comprises more than 90% by weight of a polyethylene and the outer layer (1) comprises more than 90% by weight of an aromatic or aliphatic polyether based polyurethane.

4. The tube (10) of claim 1 wherein the adhesive comprises more than 90% by weight of one or more ethylene acrylic copolymers and the inner layer (3) comprises more than 90% by weight of a polyethylene and the outer layer (1) comprises more than 90% by weight of an aromatic or aliphatic polyether based polyurethane.

5. The tube (10) of claim 1 where the polyethylene comprises one or more of a low density polyethylene, a linear low density polyethylene and a high density polyethylene and wherein the aromatic polyether based polyurethane comprises a polytetramethyleneglycol-based polyurethane.

6. The tube (10) of claim 1 where the inner layer (3) comprises more than 90% by weight of low density polyethylene (LDPE), and the outer layer (1) comprises more than 90% by weight of a polytetramethyleneglycol-based polyurethane.

7. The tube (10) of claim 1 wherein the thickness of the polyurethane outer layer is between 0.001 inches (0.0254 mm) and 0.025 inches (0.635 mm), the thickness of the inner polyethylene layer is between 0.001 inches (0.0254 mm) and 0.025 inches (0.635 mm) and the thickness of the barrier layer is between 0.001 inches (0.0254 mm) and 0.025 inches (0.635 mm).

8. The tube (10) of claim 1 wherein the inner (3) and outer (1) layers do not delaminate when subjected to a stress and strain up to the tube's elastic yield point as measured in a mechanical tester at a pull rate of about 12 inches (305 mm) per minute at ambient conditions of 72 degrees F (22° C) and 50% relative humidity.

9. The tube (10) of claim 1 wherein the tube has a central axial fluid flow passage (20), the inner layer having a radially inner wall surface that defines the passage, the outer and inner layers resisting delamination when subjected to a stress and strain up to the tube's elastic yield point as measured in a mechanical tester at a pull rate of about 12 inches (305 mm) per minute at ambient conditions of 72 degrees F (22° C) and 50% relative humidity.

10. The tube (10) of claim 6 wherein thickness of the adhesive (7) disposed between the barrier layer (5) and the outer layer (1) is between 0.001 inches (0.0254 mm) and 0.025 inches (0.635 mm) and wherein the thickness of the adhesive (9) disposed between the barrier layer (5) and the inner layer (3) is between 0.001 inches (0.0254 mm) and 0.025 inches (0.635 mm).

11. Method of forming a medical tube (10) comprising an outer layer (1), an innermost layer (3) and an intermediate layer (5) disposed between the outer layer (1) and the innermost layer (3), the method comprising:
selecting a first polymeric material having a selected structural stability, wherein the first polymeric material is selected to be a polyurethane;
selecting a second polymeric material that is inert to aqueous fluids, wherein the second polymeric material is selected to be a polyethylene;
selecting a third polymeric material that acts as a barrier to gas, wherein the third polymeric material is selected from the group consisting of an ethylene vinyl alcohol copolymer and a polyamide;
selecting a fourth polymeric material that readily bonds and adheres to the first and second polymeric materials on co-extrusion and cooling of the materials, wherein the fourth polymeric material is one or more ethylene acrylic copolymers.
co-extruding the selected first, second, third and fourth polymeric materials to form the medical tubing in a configuration that has an outer layer (1) comprising at least 90% by weight of the first polymeric material, an inner layer (3) comprising at least 90% weight of the second polymeric material, a layer (5) disposed between the inner and outer layers that comprises at least 90% by weight of the third polymeric material, a layer (7) of the fourth material disposed between the outer layer and the layer of the third polymeric material (9) and a layer of the fourth material disposed between the inner layer (3) and the layer of the third polymeric material.

12. The method of claim 11 wherein the adhesive comprises one or more anhydride grafted ethylene acrylate copolymers.

## Patentansprüche

1. Schlauch (10), der eine innere Schicht (3), eine äußere Schicht (1) und eine Barriereschicht (5), die zwischen der inneren Schicht (3) und der äußeren Schicht (1) angeordnet ist, umfasst, wobei die Barriereschicht (5) an die äußere Schicht (1) durch eine Klebstoffschicht (7), die zwischen der äußeren Schicht (1) und der Barriereschicht (5) angeordnet ist, gebunden ist, wobei die innere Schicht (3) ein Polyethylen umfasst, die äußere Schicht (1) ein thermoplastisches Polyurethan umfasst und die Barriereschicht (5) ein Material umfasst, das als eine Barriere gegenüber Gas wirkt, **dadurch gekennzeichnet, dass** die Barriereschicht (5) an die innere Schicht (3) durch eine Klebstoffschicht (9), die zwischen der inneren Schicht (3) und der Barriereschicht (5) angeordnet ist, gebunden ist, die Barriereschicht (5) mehr als 90 Gew.-% eines Ethylen-Vinylalkohol-Copolymers oder eines Polyamids oder von Blends davon umfasst und der Klebstoff der Klebstoffschichten (7, 9) ein oder mehrere Ethylen-Acrylsäure-Copolymere umfassen.

2. Schlauch (10) nach Anspruch 1, wobei der Klebstoff ein oder mehrere anhydridgepfropfte Ethylen-AcrylatCopolymere umfasst, gegebenenfalls wobei der Klebstoff ein oder mehrere anhydridgepfropfte Ethylen-Methylacrylat-Copolymere umfasst oder alternativ dazu wobei der Klebstoff mehr als 90 Gew.-% eines oder mehrerer Ethylen-Acrylsäure-Copolymere umfasst.

3. Schlauch (10) nach Anspruch 1, wobei die innere Schicht (3) mehr als 90 Gew.-% eines Polyethylens umfasst und die äußere Schicht (1) mehr als 90 Gew.-% eines Polyurethans auf der Basis von aromatischem oder aliphatischem Polyether umfasst.

4. Schlauch (10) nach Anspruch 1, wobei der Klebstoff mehr als 90 Gew.-% eines oder mehrerer Ethylen-Acrylsäure-Copolymere umfasst und die innere Schicht (3) mehr als 90 Gew.-% eines Polyethylens umfasst und die äußere Schicht (1) mehr als 90 Gew.-% eines Polyurethans auf der Basis von aromatischem oder aliphatischem Polyether umfasst.

5. Schlauch (10) nach Anspruch 1, wobei das Polyethylen eines oder mehrere von einem Polyethylen niedriger Dichte, einem linearen Polyethylen niedriger Dichte und einem Polyethylen hoher Dichte umfasst und wobei das Polyethylen auf der Basis von aromatischem Polyether ein Polyurethan auf der Basis von Polytetramethylenglykol umfasst.

6. Schlauch (10) nach Anspruch 1, wobei die innere Schicht (3) mehr als 90 Gew.-% Polyethylen niedriger Dichte (LDPE) umfasst und die äußere Schicht (1) mehr als 90 Gew.-% eines Polyurethans auf der Basis von Polytetramethylenglykol umfasst.

7. Schlauch (10) nach Anspruch 1, wobei die Dicke der äußere Polyurethanschicht zwischen 0,001 Zoll (0,0254 mm) und 0,025 Zoll (0,635 mm) liegt, die Dicke der inneren Polyethylenschicht zwischen 0,001 Zoll (0,0254 mm) und 0,025 Zoll (0,635 mm) liegt und die Dicke der Barriereschicht zwischen 0,001 Zoll (0,0254 mm) und 0,025 Zoll (0,635 mm) liegt.

8. Schlauch (10) nach Anspruch 1, wobei die innere Schicht (3) und die äußere Schicht (1) nicht delaminieren, wenn sie einer Spannung und Dehnung bis zur Dehngrenze des Schlauchs unterzogen werden, wie in einem mechanischen Prüfgerät bei einer Zuggeschwindigkeit von etwa 12 Zoll (305 mm) pro Minute bei Umgebungsbedingungen von 72 Grad F (22 °C) und 50 % relativer Luftfeuchtigkeit gemessen.

9. Schlauch (10) nach Anspruch 1, wobei der Schlauch einen zentralen axialen Flüssigkeitsfließweg (20) aufweist, wobei die innere Schicht eine radiale Innenwandoberfläche aufweist, die den Weg definiert, wobei die äußere und die innere Schicht einer Delaminierung widerstehen, wenn sie einer Spannung und Dehnung bis zur Dehngrenze des Schlauchs unterzogen werden, wie in einem mechanischen Prüfgerät bei einer Zuggeschwindigkeit von etwa 12 Zoll (305 mm) pro Minute bei Umgebungsbedingungen von 72 Grad F (22 °C) und 50 % relativer Luftfeuchtigkeit gemessen.

10. Schlauch (10) nach Anspruch 6, wobei die Dicke des Klebstoffs (7), der zwischen der Barriereschicht (5) und der äußeren Schicht (1) angeordnet ist, zwischen 0,001 Zoll (0,0254 mm) und 0,025 Zoll (0,635 mm) liegt und wobei die Dicke des Klebstoffs (9), der zwischen der Barriereschicht (5) und der inneren Schicht (3) angeordnet ist, zwischen 0,001 Zoll (0,0254 mm) und 0,025 Zoll (0,635 mm) liegt.

11. Verfahren zur Herstellung eines medizinischen Schlauchs (10), der eine äußere Schicht (1), eine innerste Schicht (3) und eine Zwischenschicht (5), die zwischen der äußere Schicht (1) und der innersten Schicht (3) angeordnet ist, umfasst, wobei das Verfahren Folgendes umfasst:
Auswählen eines ersten polymeren Materials mit einer ausgewählten strukturellen Stabilität, wobei das erste polymere Material als ein Polyurethan ausgewählt ist;
Auswählen eines zweiten polymeren Materials, das gegenüber wässrigen Flüssigkeiten inert ist, wobei das zweite polymere Material als ein Polyethylen ausgewählt ist;
Auswählen eines dritten polymeren Materials, das als eine Barriere gegenüber Gas wirkt, wobei das dritte polymere Material aus der Gruppe bestehend aus einem Ethylen-Vinylalkohol-Copolymer und einem Polyamid ausgewählt ist;
Auswählen eines vierten polymeren Materials, das sich leicht mit dem ersten und dem zweiten polymeren Material bei Koextrusion und Abkühlen der Materialien bindet und an diesen haftet, wobei es sich bei dem vierten polymeren Material um ein oder mehrere Ethylen-Acrylsäure-Copolymere handelt;
Koextrudieren des ausgewählten ersten, zweiten, dritten und vierten polymeren Materials, um das medizinische Schlauchmaterial in einer Konfiguration herzustellen, die eine äußere Schicht (1), die mindestens 90 Gew.-% des ersten polymeren Materials umfasst, eine innere Schicht (3), die mindestens 90 Gew.-% des zweiten polymeren Materials umfasst, eine Schicht (5), die zwischen der inneren und der äußeren Schicht angeordnet ist und die mindestens 90 Gew.-% des dritten polymeren Materials umfasst, eine Schicht (7) aus dem vierten Material, die zwischen der äußeren Schicht und der Schicht aus dem dritten polymeren Material (9) angeordnet ist, und eine Schicht aus dem vierten Material, die zwischen der inneren Schicht (3) und der Schicht aus dem dritten polymeren Material angeordnet ist, aufweist.

12. Verfahren nach Anspruch 11, wobei der Klebstoff ein oder mehrere anhydridgepfropfte Ethylen-AcrylatCopolymere umfasst.

## Revendications

1. Un tube (10) comprenant une couche intérieure (3), une couche extérieure (1) et une couche de barrière (5) disposée entre la couche intérieure (3) et la couche extérieure (1), dans lequel la couche de barrière (5) est liée à la couche extérieure (1) par une couche d'adhésif (7) disposée entre la couche extérieure (1) et la couche de barrière (5), dans lequel la couche intérieure (3) comprend un polyéthylène, la couche extérieure (1) comprend un polyuréthane thermoplastique et la couche de barrière (5) comprend un matériau qui sert de barrière au gaz,
**caractérisé en ce que** la couche de barrière (5) est liée à la couche intérieure (3) par une couche d'adhésif (9) disposée entre la couche intérieure (3) et la couche de barrière (5), la couche de barrière (5) comprend plus de 90% en poids d'un copolymère éthylène - alcool vinylique ou d'un polyamide ou de mélanges de ces derniers, et l'adhésif des couches d'adhésif (7, 9) comprend un ou plusieurs copolymères d'éthylène - acide acrylique.

2. Le tube (10) selon la revendication 1 dans lequel l'adhésif comprend un ou plusieurs copolymères d'éthylène acrylate greffés à un anhydride, en option dans lequel l'adhésif comprend un ou plusieurs copolymères d'acrylate de méthyle - éthylène greffés à un anhydride, ou alternativement comprend plus de 90% en poids d'un ou de plusieurs copolymères d'éthylène - acide acrylique.

3. Le tube (10) selon la revendication 1 dans lequel la couche intérieure (3) comprend plus de 90% en poids d'un polyéthylène et la couche extérieure (1) comprend plus de 90% en poids d'un polyuréthane à base de polyéther aromatique ou aliphatique.

4. Le tube (10) selon la revendication 1 dans lequel l'adhésif comprend plus de 90% en poids d'un ou de plusieurs copolymères d'éthylène - acide acrylique et la couche intérieure (3) comprend plus de 90% en poids d'un polyéthylène et la couche extérieure (1) comprend plus de 90% en poids d'un polyuréthane à base de polyéther aromatique ou aliphatique.

5. Le tube (10) selon la revendication 1 dans lequel le polyéthylène comprend un ou plusieurs parmi un polyéthylène basse densité, un polyéthylène basse densité linéaire et un polyéthylène haute densité et dans lequel le polyuréthane à base de polyéther aromatique comprend un polyuréthane à base de polytétraméthylène glycol.

6. Le tube (10) selon la revendication 1 dans lequel la couche intérieure (3) comprend plus de 90% en poids of polyéthylène basse densité (LDPE) et la couche extérieure (1) comprend plus de 90% en poids d'un polyuréthane à base de polytétraméthylène glycol.

7. Le tube (10) selon la revendication 1 dans lequel l'épaisseur de la couche extérieure en polyuréthane (1) est comprise entre 0,001 pouce (0,0254 mm) et 0,025 pouce (0,635 mm), l'épaisseur de la couche de polyéthylène intérieure est comprise entre 0,001 pouce (0,0254 mm) et 0,025 pouce (0,635 mm) et l'épaisseur de la couche de barrière est comprise entre 0,001 pouce (0,0254 mm) et 0,025 pouce (0,635 mm).

8. Le tube (10) selon la revendication 1 dans lequel les couches intérieure (3) et extérieure (1) ne se séparent pas lorsqu'elles sont soumises à une contrainte et une déformation jusqu'à la limite d'élasticité du tube telle que mesurée dans un appareil d'essai mécanique à une vitesse de tirage d'environ 12 pouces (305 mm) par minute dans les conditions ambiantes de 72 degrés F (22°C) et de 50% d'humidité relative.

9. Le tube (10) selon la revendication 1 dans lequel le tube a un passage d'écoulement fluidique axial central (20), la couche intérieure ayant une surface de paroi interne radiale qui définit le passage, les couches extérieure et intérieure résistant à la séparation lorsqu'elles sont soumises à une contrainte et une déformation jusqu'à la limite d'élasticité du tube telle que mesurée dans un appareil d'essai mécanique à une vitesse de tirage d'environ 12 pouces (305 mm) par minute dans les conditions ambiantes de 72 degrés F (22°C) et de 50% d'humidité relative.

10. Le tube (10) selon la revendication 6 dans lequel l'épaisseur de l'adhésif (7) disposé entre la couche de barrière (5) et la couche extérieure (1) est comprise entre 0,001 pouce (0,0254 mm) et 0,025 pouce (0,635 mm) et dans lequel l'épaisseur de l'adhésif (9) disposé entre la couche de barrière (5) et la couche intérieure (3) est comprise entre 0,001 pouce (0,0254 mm) et 0,025 pouce (0,635 mm).

11. Un procédé permettant de former un tube médical (10) comprenant une couche extérieure (1), une couche la plus intérieure (3) et une couche intermédiaire (5) disposée entre la couche extérieure (1) et la couche la plus intérieure (3), le procédé consistant à :
sélectionner un premier matériau polymère ayant une stabilité structurelle sélectionnée, dans lequel le premier matériau polymère est sélectionné pour être un polyuréthane ;
sélectionner un deuxième matériau polymère qui est inerte aux fluides aqueux, dans lequel le deuxième matériau polymère est sélectionné pour être un polyéthylène ;
sélectionner un troisième matériau polymère qui sert de barrière au gaz, dans lequel le troisième matériau polymère est sélectionné dans le groupe constitué par un copolymère éthylène - alcool vinylique et un polyamide ;
sélectionner un quatrième matériau polymère qui se colle facilement et adhère aux premier et deuxième matériaux polymères lors de la co-extrusion et du refroidissement des matériaux, dans lequel le quatrième matériau polymère est un ou plusieurs copolymères d'éthylène - acide acrylique ;
co-extruder les premier, deuxième, troisième et quatrième matériaux polymères sélectionnés pour former le tube médical dans une configuration qui a une couche extérieure (1) comprenant au moins 90% en poids du premier matériau polymère, une couche intérieure (3) comprenant au moins 90% en poids du deuxième matériau polymère, une couche (5) disposée entre les couches intérieure et extérieure qui comprend au moins 90% en poids du troisième matériau polymère, une couche (7) du quatrième matériau disposée entre la couche extérieure et la couche du troisième matériau polymère (9) et une couche du quatrième matériau disposée entre la couche intérieure (3) et la couche du troisième matériau polymère.

12. Le procédé selon la revendication 11 dans lequel l'adhésif comprend un ou plusieurs copolymères d'éthylène acrylate greffés à un anhydride.
